# EUROPEAN PATENT APPLICATION

(11) **EP 3 991 657 A1**
(43) Date of publication of application: **04.05.2022**
(21) Application number: 21206004.0
(22) Date of filing: 02.11.2021
(51) Int. Cl.: A61B 5/308, A61B 5/00

(54) **ACQUISITION BOX, ACQUISITION BOX ASSEMBLY, AND MONITORING APPARATUS**

(30) Priority: 02.11.2020 CN 202011208030
(71) Applicant: Shenzhen Mindray Bio-Medical Electronics Co., Ltd., Shenzhen, Guangdong 518057 (CN)
(72) Inventor: LIU, Qiling, Shenzhen, 518057 (CN); REN, Jian, Shenzhen, 518057 (CN)
(74) Representative: KIPA AB

(57) **Abstract**

The disclosure provides an acquisition box, comprising a signal receiving end, a voltage processing module, and a wireless communication module. The signal receiving end is configured to receive a measurement signal acquired from a subject. The voltage processing module is configured to reduce a voltage of the measurement signal received by the signal receiving end to obtain a target measurement signal. The wireless communication module is configured to transmit the target measurement signal to a target monitoring device in a wireless mode. The disclosure further provides an acquisition box assembly and a monitoring apparatus. According to the disclosure, the target measurement signal is obtained by an acquisition box independent of the monitoring device to at least reduce the voltage of the measurement signal received by the signal receiving end, achieving a defibrillation protection function; the size of the target monitoring device may be kept small, and the target measurement signal is transmitted to the target monitoring device in a wireless mode, meeting the ECG measurement requirement and also bringing convenience for a user.

## Description

### TECHNICAL FIELD

The disclosure relates to the field of physiological parameter measurement, and in particular to an acquisition box, an acquisition box assembly and a monitoring apparatus for physiological parameter measurement.

### BACKGROUND

An ECG (Electrocardiograph) measurement system is configured to record an electrical activity of a heart within a period by measuring a surface potential of a living tissue. The current ECG measurement system uses a plurality of leads, such as 6 leads or 12 leads, one end of which is configured to be in contact with a subject, and the other end of which is connected to an ECG processing module, so that ECG signals are provided to the ECG processing module, achieving measurement of the ECG signals.

When a patient needs a defibrillation therapy or needs to receive a surgical therapy using an electrotome, if a lead is still connected to the patient to monitor heartbeat information of the patient in real time, a high voltage, a strong current, etc. conducted by the lead during the defibrillation or electrotome therapy may damage the ECG measurement system or affect measurement data. Therefore, it is required to design a defibrillation protection circuit/anti-defibrillation circuit for the ECG measurement system to reduce or clamp the voltage in order to avoid the above problems. Taking the defibrillation protection circuit as an example, it is necessary to ensure that the defibrillation energy absorbed by the ECG processing module is less than standard requirements and to protect the ECG processing module from being damaged by the defibrillation energy.

Therefore, how to design an ECG measurement system/apparatus having a defibrillation protection function to meet the above requirements, and especially for a wearable ECG measurement apparatus, how to avoid affecting the ECG measurement system during a defibrillation therapy while keeping the apparatus miniaturized becomes a problem to be solved.

### SUMMARY

An embodiment of the disclosure provides an acquisition box, an acquisition box assembly and a monitoring apparatus for physiological parameter measurement in order to solve the above problems.

In an aspect, provided is an acquisition box. The acquisition box includes a signal receiving end, a voltage processing module, and a wireless communication module. The signal receiving end is configured to receive a measurement signal acquired from a subject. The voltage processing module is configured to reduce a voltage of the measurement signal received by the signal receiving end to obtain a target measurement signal. The wireless communication module is configured to transmit the target measurement signal to a target monitoring device in a wireless mode.

In another aspect, provided is an acquisition box assembly. The acquisition box assembly includes an acquisition box and a measurement wire, wherein the measurement wire is detachably or fixedly connected to the acquisition box, and the measurement wire is configured to acquire a measurement signal from a subject; and the acquisition box includes a signal receiving end, a voltage processing module and a wireless communication module. The signal receiving end is configured to receive a measurement signal acquired from a subject. The voltage processing module is configured to reduce a voltage of the measurement signal received by the signal receiving end to obtain a target measurement signal. The wireless communication module is configured to transmit the target measurement signal to a target monitoring device in a wireless mode.

In a further aspect, provided is a monitoring apparatus. The monitoring apparatus includes a monitoring device and an acquisition box, the monitoring device and the acquisition box being connected in a wireless communication mode. The acquisition box includes a signal receiving end, a voltage processing module and a wireless communication module. The signal receiving end is configured to receive a measurement signal acquired from a subject. The voltage processing module is configured to reduce a voltage of the measurement signal received by the signal receiving end to obtain a target measurement signal. The wireless communication module is configured to transmit the target measurement signal to a target monitoring device in a wireless mode.

According to the disclosure, the target measurement signal is obtained by an acquisition box independent of the monitoring device to at least reduce the voltage of the measurement signal received by the signal receiving end, achieving a defibrillation protection function; the size of the target monitoring device may be kept small, and the target measurement signal is transmitted to the target monitoring device in a wireless mode, meeting the ECG measurement requirement and also bringing convenience for a user.

### BRIEF DESCRIPTION OF THE DRAWINGS

In order to more clearly describe the technical solutions in the embodiments of the disclosure or in the prior art, the accompanying drawings required for describing the embodiments or the prior art will be briefly described below. Apparently, the accompanying drawings in the following description show only some of the embodiments of the disclosure, and those of ordinary skill in the art may still derive other drawings from these drawings without involving any inventive effort.
FIG. 1 is a structural block diagram of an acquisition box according to an embodiment of the disclosure.
FIG. 2 is a detailed structural block diagram of the acquisition box according to an embodiment of the disclosure.
FIG. 3 is a top view of part of an internal structure of the acquisition box according to an embodiment of the disclosure.
FIG. 4 is a cross-sectional view of the acquisition box according to an embodiment of the disclosure.
FIG. 5 is a cross-sectional view of the acquisition box according to another embodiment of the disclosure.
FIG. 6 is a cross-sectional view of the acquisition box according to a further embodiment of the disclosure.
FIG. 7 is a top view of part of the internal structure of the acquisition box according to other embodiment of the disclosure.
FIG. 8 is a detailed schematic structural diagram of a voltage processing module according to an embodiment of the disclosure.
FIG. 9 is a detailed schematic top view of part of the internal structure of the acquisition box according to an embodiment of the disclosure.
FIG. 10 is a schematic diagram of an acquisition box assembly according to an embodiment of the disclosure.
FIG. 11 is a schematic diagram of a monitoring apparatus according to an embodiment of the disclosure.
FIG. 12 is a schematic diagram of the monitoring apparatus according to another embodiment of the disclosure.
FIG. 13 is a schematic diagram of the monitoring apparatus worn on a subject according to an embodiment of the disclosure.

### DESCRIPTION OF THE PREFERRED EMBODIMENTS

To make the solutions of the disclosure more comprehensible to those skilled in the art, the technical solutions of the embodiments of the disclosure will be described below clearly and comprehensively in conjunction with the accompanying drawings of the embodiments of the disclosure. Clearly, the embodiments described are merely some embodiments of the disclosure and are not all the possible embodiments. Based on the embodiments in the disclosure, all the other embodiments that would have been obtained by those of ordinary skill in the art without any inventive effort shall fall within the scope of protection of the disclosure.

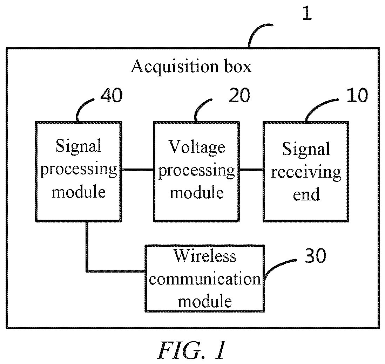

Referring to FIG. 1, it is a structural block diagram of an acquisition box 1 according to an embodiment of the disclosure. As shown in FIG. 1, the acquisition box 1 includes a signal receiving end 10, a voltage processing module 20, and a wireless communication module 30. The signal receiving end 10 is configured to receive a measurement signal acquired from a subject; the voltage processing module 20 is configured to at least reduce a voltage of the measurement signal received by the signal receiving end 10 to obtain a target measurement signal; and the wireless communication module 30 is configured to transmit the target measurement signal to a target monitoring device 2 (also referred to herein as a monitoring device 2) in a wireless mode.

Thus, according to the disclosure, the target measurement signal is obtained by an acquisition box 1 independent of the monitoring device 2 to at least reduce the voltage of the measurement signal received by the signal receiving end 10, achieving a defibrillation protection function; the size of the target monitoring device 2 may be kept small, and the target measurement signal is transmitted to the target monitoring device 2 in a wireless mode, meeting the ECG measurement requirement and also bringing convenience for the user. It should be understood that the voltage processing module 20 in each embodiment of the disclosure is configured to reduce the voltage of the measurement signal received by the signal receiving end 10 so as to obtain the target measurement signal, but the voltage processing module 20 reduces the voltage of the received measurement signal only when the measurement signal received by the signal receiving end 10 includes a high voltage signal, for example, when a patient undergoes a defibrillation therapy or a surgical therapy using an electrotome. However, in other conventional cases, that is, when the patient does not receive a defibrillation therapy or a surgical therapy using an electrotome, etc., if the measurement signal does not include a high voltage signal, there is no need to reduce the voltage via the voltage processing module 20.

The signal receiving end 10, the voltage processing module 20 and the wireless communication module 30 are sequentially connected.

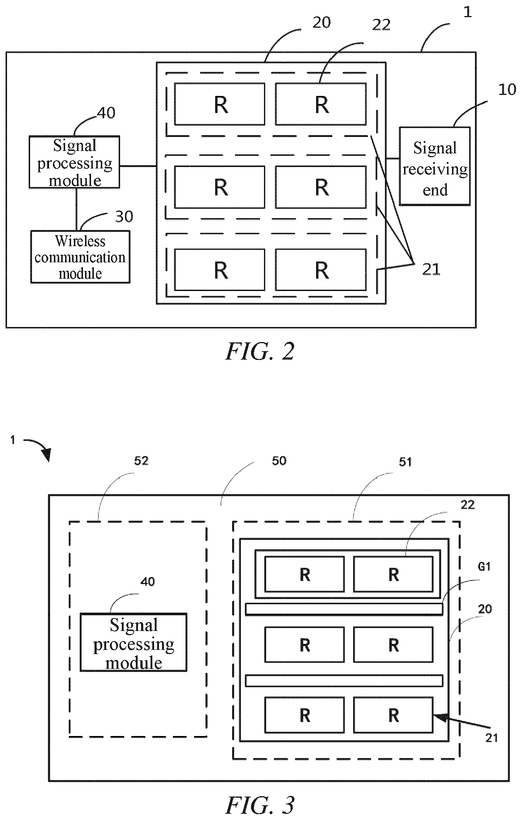

Referring to FIG. 2, it is a more detailed structural block diagram of the acquisition box 1 according to an embodiment of the disclosure. As shown in FIG. 2, the voltage processing module 20 includes an anti-defibrillation resistor module 21. The anti-defibrillation resistor module includes an anti-defibrillation resistor 22 (further indicated with a symbol R in FIG. 2), the anti-defibrillation resistor 22 is connected to the signal receiving end 10, and the measurement signal received by the signal receiving end 10 passes through the anti-defibrillation resistor 22 to reduce the voltage.

That is, the anti-defibrillation resistor 22 is configured to reduce voltage of the measurement signal received by the signal receiving end 10. After the measurement signal received by the signal receiving end 10 runs through the anti-defibrillation resistor 22, the voltage is reduced by means of voltage dividing of the anti-defibrillation resistor 22.

Thus, when a high voltage signal such as a high voltage for defibrillation is included in the measurement signal, the high voltage signal such as the high voltage for defibrillation will be reduced to a smaller voltage after running through the anti-defibrillation resistor module 21/the anti-defibrillation resistor 22 in the voltage processing module 20, which does not affect EGC measurement or greatly reduces the negative effect on the EGC measurement.

As shown in FIG. 2, the signal receiving end 10 is specifically configured to receive a plurality of measurement signals, the anti-defibrillation resistor module 21 includes a plurality of groups of anti-defibrillation resistor(s) 22, and each of the measurement signals passes through a corresponding group of anti-defibrillation resistor(s) 22 to reduce the voltage.

The number of the groups of defibrillation resistor(s) 22 may be greater than or equal to the number of the plurality of measurement signals, and when the signal receiving end 10 receives the plurality of measurement signals, each of the measurement signals is transmitted by means of a corresponding group of anti-defibrillation resistor(s) 22, and the voltage is reduced by means of the corresponding group of anti-defibrillation resistor(s) 22.

Each group of anti-defibrillation resistor(s) 22 includes at least one anti-defibrillation resistor connected in series, and different groups of anti-defibrillation resistor(s) include the same number or different numbers of anti-defibrillation resistor(s).

In some embodiments, the signal receiving end 10 of the acquisition box 1 may be detachably or fixedly connected to a straight or bifurcated measurement wire/lead, and a plurality of electrode pads may be connected to the measurement wire and are in contact with different parts of the subject to obtain the plurality of measurement signals. The signal receiving end 10 of the acquisition box 1 is connected to the straight or bifurcated measurement wire/lead to obtain the plurality of measurement signals.

As shown in FIGS. 1 and 2, the acquisition box 1 further includes a signal processing module 40, the signal processing module 40 is coupled to the voltage processing module 20 and configured to perform analog-to-digital conversion on the measurement signal that has passed through the voltage processing module 20 to reduce the voltage, and the target measurement signal is a digital measurement signal that has undergone analog-to-digital conversion. That is, the target measurement signal transmitted to the target monitoring device 2 by the wireless communication module 30 in a wireless mode is the digital measurement signal obtained by running through the above-mentioned voltage processing module 20 to reduce the voltage and then by performing analog-to-digital conversion on the measurement signal with the reduced voltage.

Referring to FIG. 3, it is a top view of part of an internal structure of the acquisition box 1 according to an embodiment of the disclosure. As shown in FIG. 3, the acquisition box 1 further includes a circuit board 50, the circuit board 50 including a first region 51 and a second region 52. The voltage processing module 20 is disposed in the first region 51 of the circuit board 50, the signal processing module 40 is disposed in the second region 52 of the circuit board 50, and the first region 51 is located between the second region 52 and the signal receiving end 10.

The first region 51 and the second region 52 are two regions which do not overlap.

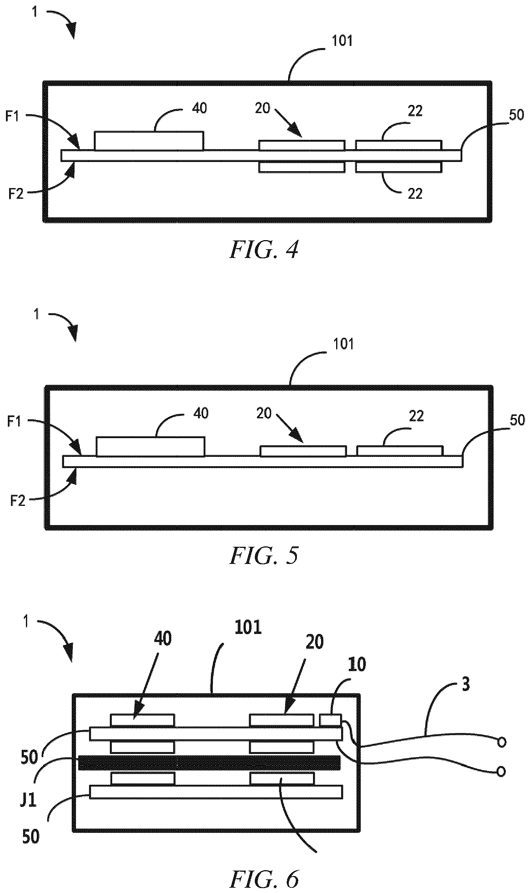

Referring to FIG. 4, it is a cross-sectional view of the acquisition box 1 according to an embodiment of the disclosure. The circuit board 50 includes two faces opposing from each other, a group of elements of the voltage processing module 20 are disposed on a face of the first region 51 of the circuit board, another group of elements are disposed on the other face of the first region 51 of the circuit board 50, and the signal processing module 40 is disposed on at least one face of the second region 52 of the circuit board 50.

Specifically, the circuit board 50 includes an upper surface F1 and a lower surface F2 that oppose from each other, and at least some of the groups of anti-defibrillation resistor(s) 22 included in the voltage processing module 20 are disposed on the upper surface F1 of the first region 51 of the circuit board 50, and other groups of anti-defibrillation resistor(s) 22 are disposed on the upper surface F1 of the first region 51 of the circuit board 50. Alternatively, in some embodiments, a group of elements of the signal processing module 40 are also disposed on the upper surface F1 of the second region 52 of the circuit board 50, and another group of elements are disposed on the lower surface F2 of the second region 52 of the circuit board 50.

Referring to FIG. 5, it is a cross-sectional view of the acquisition box 1 according to another embodiment of the disclosure. As shown in FIG. 5, in another embodiment, the voltage processing module 20 and the signal processing module 40 are disposed on the same face of the circuit board 50.

That is, in another embodiment, all elements included in the voltage processing module 20 and the signal processing module 40 are located on the same face of the circuit board 50. Therefore, the voltage processing module 20 and the signal processing module 40 are located in the first region 51 and the second region 52 of the circuit board 50 respectively, and are flatly laid on the same face of the circuit board 50.

Both the voltage processing module 20 and the signal processing module 40 may be disposed on the upper surface F1 of the circuit board 50 or on the lower surface F2 of the circuit board 50.

Referring to FIG. 6, it is a cross-sectional view of the acquisition box 1 according to a further embodiment of the disclosure. As shown in FIG. 6, in a further embodiment, the acquisition box 1 includes at least two circuit boards 50, that is, at least two circuit boards 50 are provided. The at least two circuit boards 50 are disposed in a stacked manner, each circuit board 50 includes a first region 51 and a second region 52. A group of elements of the voltage processing module 20 and a group of elements of the signal processing module 40 are respectively disposed in the first region 51 and the second region 52 of one circuit board 50; another group of elements of the voltage processing module 20 and another group of elements of the signal processing module 40 are respectively disposed in the corresponding first region 51 and the corresponding second region 52 of the other circuit board 50.

Thus, by means of the stacking configuration, the space of the acquisition box 1 may be effectively utilized, and the sizes of the acquisition box 1 in lengthwise and widthwise directions may be reduced.

When the acquisition box 1 includes at least two circuit boards 50 and the at least two circuit boards 50 are disposed in a stacked manner, at least one face of each circuit board 50 is provided with elements of the signal processing module 40 and the voltage processing module 20, and two faces of at least one circuit board 50 are both provided with elements of the voltage processing module 20.

For example, as shown in FIG. 6, the acquisition box 1 includes two circuit boards 50, where the upper surface F1 of one circuit board 50 is provided with the elements of the signal processing module 40 and the voltage processing module 20, the upper surface F2 of the other circuit board 50 is also provided with the elements of the signal processing module 40 and the voltage processing module 20, and in the first region 51 of the lower surface of the other circuit board 50, the elements of the voltage processing module 20 are also provided.

As shown in FIG. 6, an insulating material J1 is disposed between two adjacent circuit boards 50. The insulating material J1 is configured to electrically isolate every two adjacent circuit boards 50 of the at least two stacked circuit boards 50.

The insulating material J1 may be an insulating material such as plastic or resin. As shown in FIG. 6, the insulating material J1 may be formed in a layered/sheet structure and may extend completely between two adjacent circuit boards 50, that is, a projection of the insulating material J1 on a circuit board 50 may coincide with the circuit board 50. Obviously, the insulating material J1 may also extend only in the region where the elements of the signal processing module 40 and the voltage processing module 20 are provided.

As shown in FIGS. 4-6, the acquisition box 1 further includes a housing 101, the above-mentioned circuit boards 50 being located in the housing 101. The insulating material J1 may be epoxy resin, etc., and the insulating material J1 may be filled between every two circuit boards 50 and between the circuit board 50 and an inner wall of the housing 101. In some embodiments, the housing 101 may be filled with the insulating material J1, and the housing 101 may be filled with the epoxy resin by vacuumizing an internal space of the housing 101 and then sucking the epoxy resin in a glue state with a negative pressure in the housing 101, achieving encapsulation and effective insulation of circuit elements included in the acquisition box 1, ensuring the filling integrity and consistency, and achieving miniaturization of the acquisition box 1. Of course, in other embodiments, after the insulating material J1 encapsulates all circuit elements including the circuit board 50 inside the acquisition box 1, the insulating material J1 may directly form the housing 101.

The voltage processing module 20 and the signal receiving end 10 may be electrically connected by means of an electrical connector such as a conductive wire or a flexible circuit board, and the signal processing module 40 and the voltage processing module 20 may also be electrically connected by means of an electrical connector such as a conductive wire or a flexible circuit board.

When two faces of a certain circuit board 50 are both provided with the elements of the voltage processing module 20 and/or the signal processing module 40, in case of need, the elements of the voltage processing module 20 and/or the signal processing module 40 disposed on the two faces of the circuit board 50 may also be electrically connected through conductive holes penetrating the two faces of the circuit board 50.

For example, when two faces of a certain circuit board 50 are provided with different elements of the signal processing module 40, the different elements of the signal processing module 40 disposed on the two faces of the circuit board 50 may be electrically connected through conductive holes penetrating the two faces of the circuit board 50.

The above-mentioned signal receiving end 10 may be an interface embedded in a side wall of the housing 101, and then electrically connected to the voltage processing module 20 disposed on the circuit board 50 by means of a conductive wire, a flexible circuit board, etc. Alternatively, the signal receiving end 10 may also be disposed on the circuit board 50 in the housing 101. When the signal receiving end 10 is disposed on the circuit board 50 in the housing 101, the signal receiving end 10 may be disposed at a side end of the circuit board 50 or may also be disposed in a third region (not shown in the figures) of the circuit board 50, where the side end or the third region can be a side of the first region 51 away from the second region 52 such that the voltage processing module 20 is located between the signal receiving end 10 and the signal processing module 40.

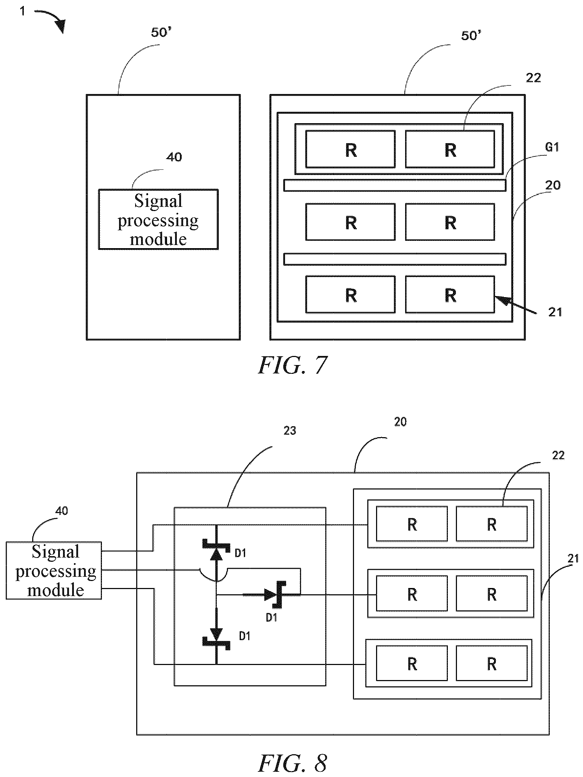

Referring to FIG. 7, it is a top view of part of the internal structure of the acquisition box 1 according to other embodiment of the disclosure. As shown in FIG. 7, the acquisition box 1 includes at least two circuit boards 50', the at least two circuit boards 50' are flatly laid, and the voltage processing module 20 and the signal processing module 40 are disposed on different circuit boards 50'.

That is, in other embodiment, the voltage processing module 20 and the signal processing module 40 are disposed on different circuit boards 50' respectively.

Obviously, the plurality of different circuit boards 50' may be connected by means of flexible circuit boards, conductive wires, etc. to form a large circuit board. Alternatively, the plurality of different circuit boards 50' may be a plurality of circuit boards formed by logically dividing the above-mentioned circuit board 50, the circuit board 50' provided with the voltage processing module 20 is a circuit board 50' at least including the above-mentioned first region 51, and the circuit board 50' provided with the signal processing module 40 is a circuit board 50' at least including the above-mentioned second region 52.

In some embodiments, when the acquisition box 1 includes at least two flatly laid circuit boards 50 or at least two stacked circuit boards 50, the signal receiving end 10 may also be disposed on an independent circuit board 50'/50, or the signal receiving end 10 may also be located at a side end of the circuit board 50'/50 provided with the voltage processing module 20 or a certain region on the circuit board 50'/50 provided with the voltage processing module 20.

Referring back to FIG. 3, in some embodiments, when two faces of a circuit board 50 are both provided with elements of the voltage processing module 20, that is, the two faces are both provided with anti-defibrillation resistor(s) 22, adjacent groups of anti-defibrillation resistor(s) 22 disposed on the same face of the same circuit board 50 are isolated.

An isolation region G1 is disposed between adjacent groups of anti-defibrillation resistor(s) 22 disposed on the same face of the same circuit board 50. Specifically, the region between the adjacent groups of anti-defibrillation resistor(s) 22 disposed on the same face of the same circuit board 50 is hollowed out, and/or the region between the adjacent groups of anti-defibrillation resistor(s) 22 disposed on the same face of the same circuit board 50 is provided with an isolation wall for isolation. The isolation region G1 may include a region formed by means of said hollowing out, and/or include the isolation wall.

The hollowing out of the region between the adjacent groups of anti-defibrillation resistor(s) 22 disposed on the same face of the same circuit board 50 may include: hollowing out the face provided with the anti-defibrillation resistor(s) 22 by a predetermined depth, and removing a metal conductive layer structure of the circuit board 50 in a predetermined region between the adjacent groups of the anti-defibrillation resistor(s) 22 on one side of the face.

The predetermined region may be a straight strip-shaped region located between the adjacent groups of anti-defibrillation resistor(s) 22, an "S" shaped region located between the adjacent groups of anti-defibrillation resistor(s) 22, etc.

The isolation wall may be made of an insulating voltage-resistant material, for example, an isolation wall made of a PET material. Generally, a material having a voltage resistance of more than 5 kV can meet the requirements, but the specific selection of an insulating voltage-resistant material depends on the voltage intensity of the measurement signal received by the signal receiving end, etc.

In some embodiments, the hollowing-out processing may also be performed, and an isolation wall may be disposed in the region between the adjacent groups of anti-defibrillation resistor(s) 22 disposed on the same face of the same circuit board 50. For example, part of the region between the adjacent groups of anti-defibrillation resistor(s) 22 is hollowed out, and part of the region is provided with an isolation wall.

In some embodiments, when an isolation wall is disposed, the isolation wall is spaced apart from the anti-defibrillation resistor(s) 22. Further, an insulating material may also be disposed between the anti-defibrillation resistor(s) 22 to insulate the resistors from each other.

The anti-defibrillation resistor 22 may be a surface-mounted resistor, and a surface-mounted resistor having a resistance meeting the requirements may be selected and welded on the circuit board 50, so that the thickness of the circuit board 50 can be reduced.

Referring to FIG. 8, it is a detailed structural diagram of the voltage processing module 20 according to an embodiment of the disclosure. As shown in FIG. 8, the voltage processing module 20 may further include a clamping circuit 23, and the clamping circuit 23 is connected to an output end of the anti-defibrillation resistor module 21 for clamping a voltage of the measurement signal to a preset voltage when the voltage of the measurement signal reduced by running through the anti-defibrillation resistor module 21 is higher than a trigger voltage.

As shown in FIG. 8, the clamping circuit 23 includes a plurality of clamping diodes D1, a negative pole of each clamping diode D1 is connected to an end, away from the signal receiving end 10, of a group of anti-defibrillation resistor(s) 22 of the anti-defibrillation resistor module 21, and positive poles of all the clamping diodes D1 are electrically connected to one another.

In some embodiments, the positive poles of all the clamping diodes D1 are electrically connected to one another and are then connected to a grounding point.

The plurality of clamping diodes D1 may be Zener diodes.

The clamping circuit 23 is located between the anti-defibrillation resistor module 21 and the signal processing module 40, such that when the voltage of the measurement signal reduced by running through the anti-defibrillation resistor module 21 is higher than the trigger voltage, the voltage of the measurement signal is clamped to the preset voltage, and the measurement signal clamped to the preset voltage is then transmitted to the signal processing module 40 for processing.

As shown in FIG. 6, the signal receiving end 10 is configured to be connected to a measurement wire 3 so as to receive a measurement signal acquired by the measurement wire 3 from the subject. As previously mentioned, the measurement wire 3 may be a straight measurement wire or a bifurcated measurement wire. The signal receiving end 10 may be detachably or fixedly connected to the straight or bifurcated measurement wire 3/lead, and a plurality of electrode pads may be connected to the measurement wire and are in contact with different parts of the subject to obtain the plurality of measurement signals. The signal receiving end 10 of the acquisition box 1 is connected to the straight or bifurcated measurement wire/lead to obtain the plurality of measurement signals.

The measurement wire 3 includes a plurality of measurement ends that are configured to respectively acquire a plurality of measurement signals from the different parts of the subject. Specifically, an electrode pad may be connected to each measurement end, and each measurement end is attached to the skin of different parts of the subject by means of the electrode pad, and acquires a corresponding measurement signal by means of the electrode pad. When the measurement wire 3 is a straight measurement wire, the measurement ends are ends of branch wires extending from different positions of the measurement wire 3. When the measurement wire 3 is a bifurcated measurement wire, the measurement wire 3 includes a main wire and a plurality of branch wires connected to the main wire by means of connectors, and the end of each branch wire away from the connector is the measurement end.

In some embodiments, when the signal receiving end 10 is an interface embedded in the side wall of the housing 101, the measurement wire 3 may be in pluggable connection with the signal receiving end 10 by means of a connector to achieve detachable connection. In other embodiments, when the signal receiving end 10 is disposed on the circuit board 50 within the housing 101, an end of the measurement wire 3 away from the measurement end may pass through the housing 101 to be fixedly connected to the signal receiving end 10 disposed on the circuit board 50 within the housing 101.

In the acquisition box of claim 1, the target monitoring device 2 includes at least one of a wearable monitoring device, a bedside monitoring device, a department-level workstation device, and a hospital-level data center/hospital-level emergency center management device.

The wireless communication module 30 includes at least one of a Bluetooth module, a WMTS communication module, an NFC communication module, a WIFI communication module, a 4G communication module and a 5G communication module.

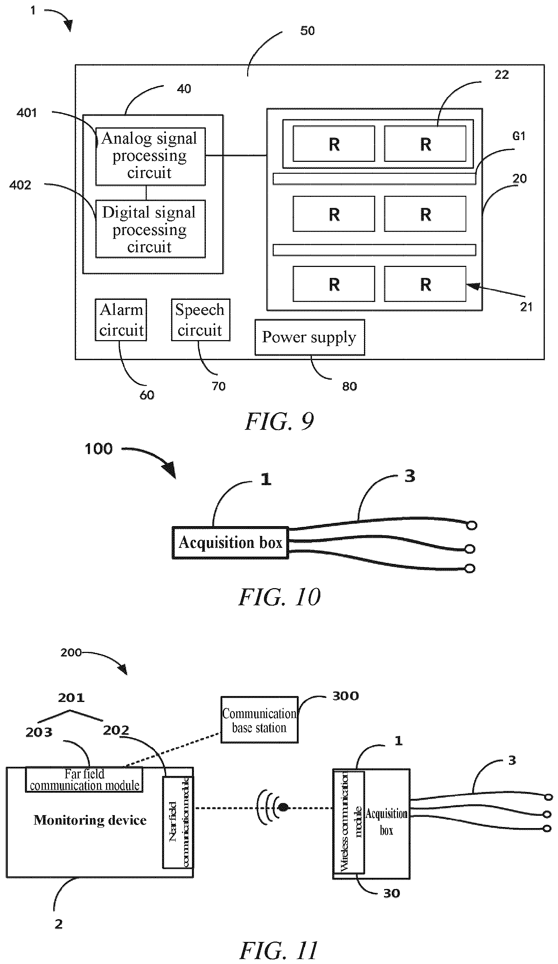

Referring to FIG. 9, it is a detailed top view of part of the internal structure of the acquisition box 1 according to an embodiment of the disclosure. As shown in FIG. 9, the signal processing module 40 specifically includes an analog signal processing circuit 401 and a digital signal processing circuit 402, the analog signal processing circuit 401 being connected between the voltage processing module 20 and the digital signal processing circuit 402. The analog signal processing circuit 401 is configured to perform analog-to-digital conversion on the measurement signal with a voltage reduced by the voltage processing module 20 to obtain a measurement signal in a digital form, and the digital signal processing circuit 402 is configured to perform further analysis processing on the measurement signal in the digital form, such as denoising processing. The analog signal processing circuit 401 and the digital signal processing circuit 402 may be disposed on the same face of the same circuit board 50 or may be disposed on two faces of the same circuit board 50 respectively, or when the circuit boards 50 are at least two stacked circuit boards 50, the analog signal processing circuit 401 and the digital signal processing circuit 402 may be disposed in the second regions 52 of different circuit boards 50 respectively.

As shown in FIG. 9, in some embodiments, the acquisition box 1 further includes an alarm circuit 60, the alarm circuit 60 being connected to the signal processing module 40, for example, specifically connected to the digital signal processing circuit 402. The signal processing module 40 may also be configured to control the alarm circuit 60 to give an alarm when an abnormality is detected, for example, the signal processing module 40 determines, when the number of received measurement signals is less than a preset number, that the measurement end of the measurement wire 3 falls off and generates an alarm signal.

The alarm circuit 60 may be a vibrator, a buzzer, etc.

As shown in FIG. 9, the acquisition box 1 may further include a speech circuit 70, the speech circuit 70 is configured to output speech information, and the speech circuit 70 is also connected to the signal processing module 40, for example, specifically connected to the digital signal processing circuit 402. The signal processing module 40 may also control the speech circuit 70 to output a speech signal under certain conditions, for example, at a predetermined measurement moment, the speech circuit 70 may output a prompt speech for prompting a user to lie flat or sit still, thereby ensuring the accuracy of measurement.

In some embodiments, the signal processing module 40 may also control the speech circuit 70 to output a speech alarm signal when an abnormality is detected.

As shown in FIG. 9, the acquisition box 1 further includes a power supply 80, and the power supply is configured to supply power to circuit elements in the acquisition box 1, for example, provide power to the signal processing module 40, the wireless communication module 30, etc. The power supply 80 may be a rechargeable battery such as a lithium battery.

The alarm circuit 60, the speech circuit 70 and the power supply 80 are all disposed on the circuit board 50.

Referring to FIG. 10, it is a schematic diagram of an acquisition box assembly 100 according to an embodiment of the disclosure. The acquisition box assembly 100 includes the above-mentioned acquisition box 1 and the measurement wire 3.

As previously described, the measurement wire 3 is a bifurcated measurement wire or a straight measurement wire, and includes a plurality of measurement ends configured to respectively acquire a plurality of measurement signals from different parts of the subject.

As previously described, the measurement end is configured to be connected to an electrode pad, and each measurement end is attached to the skin of different parts of the subject by means of the electrode pad, and acquires a corresponding measurement signal by means of the electrode pad.

The measurement signal acquired by each measurement end by means of the electrode pad may be a physiological parameter measurement signal of the subject, including at least one physiological parameter measurement signal of ECG (electrocardiogram), SpO2 (blood oxygen saturation), TEMP (temperature), NIBP (non-invasive blood pressure), RESP (respiratory rate) and IBP (invasive blood pressure) of the subject.

The specific structure of the acquisition box 1, which is depicted in the foregoing description, will not be described in detail herein.

Referring to FIG. 11, it is a schematic diagram of a monitoring apparatus 200 according to an embodiment of the disclosure. The monitoring apparatus 200 includes the above-mentioned acquisition box 1 (or the acquisition box assembly 100) and a monitoring device 2.

The monitoring device 2 is connected to the acquisition box 1 in a wireless communication mode.

The monitoring device 2 includes a wireless communication module 201, the wireless communication module 201 of the monitoring device 2 may establish a connection with the wireless communication module 30 of the acquisition box 1 in a wireless communication mode and then transmit and receive signals/data.

The wireless communication mode includes at least one of a Bluetooth communication mode, a WMTS communication mode, an NFC communication mode, a WIFI communication mode, a 4G communication mode, and a 5G communication mode.

In some embodiments, the monitoring device 2 is a wearable monitoring device or a bedside monitoring device. The wireless communication module 201 included in the monitoring device 2 includes a near field communication module 202 and a far field communication module 203, where the near field communication module 202 may include a communication module supporting the Bluetooth communication, the NFC communication, the WIFI communication, etc. as described above, and the far field communication module 203 may be a communication module supporting telephone network communication such as 4G communication and 5G communication.

The wireless communication module 30 of the acquisition box 1 at least includes a near field communication module, and the monitoring device 2 may specifically establish a wireless communication connection with the acquisition box 1 by means of the near field communication module 202 so as to receive a target measurement signal from the acquisition box 1. The far field communication module 203 of the monitoring device 2 may be in communication connection with a communication base station 300, and may be in communication connection with a department-level workstation device and a hospital-level data center/hospital-level emergency center management device, etc. by means of the communication base station 300. The monitoring device 2 may directly transmit a received target measurement signal to the department-level workstation device and the hospital-level data center/hospital-level emergency center management device by means of the far field communication module 203, so that the department-level workstation device and the hospital-level data center/hospital-level emergency center management device can obtain an analysis result by analysis and display corresponding analysis result data, or the monitoring device 2 may process and analyze the measurement signal to obtain analysis result data and send the analysis result data to the department-level workstation device and the hospital-level data center/hospital-level emergency center management device for displaying. Thus, medical care personnel at the department-level workstation device and the hospital-level data center/hospital-level emergency center management device can view the data.

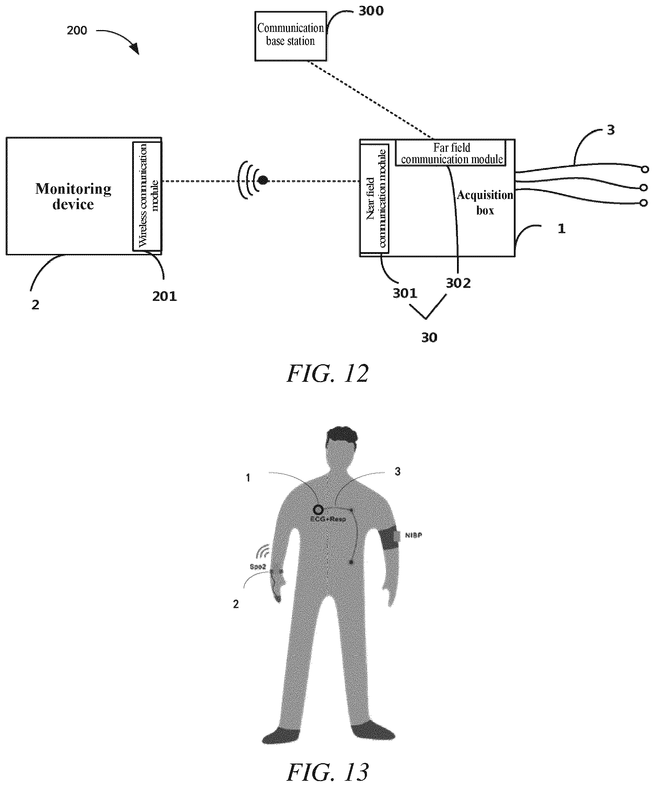

Referring to FIG. 12, it is a schematic diagram of the monitoring apparatus 200 according to another embodiment of the disclosure. The monitoring apparatus 200 includes the above-mentioned acquisition box 1 (or the acquisition box assembly 100) and a monitoring device 2. In another embodiment, the monitoring device 2 is a wearable monitoring device or a bedside monitoring device, and the wireless communication module 30 included in the acquisition box 1 includes a near field communication module 301 and a far field communication module 302. Similarly, the near field communication module 301 may include a communication module supporting the Bluetooth communication, the NFC communication, the WIFI communication, etc. as described above, and the far field communication module 302 may be a communication module supporting telephone network communication such as 4G communication and 5G communication.

The wireless communication module 201 of the monitoring device 2 at least includes a near field communication module, and the acquisition box 1 may specifically establish a wireless communication connection with the monitoring device 2 by means of the near field communication module 301 and transmit a target measurement signal to the monitoring device 2. The far field communication module 302 of the acquisition box 1 may be in communication connection with the communication base station 300, and may be in communication connection with a department-level workstation device and a hospital-level data center/hospital-level emergency center management device, etc. by means of the communication base station 300; and the acquisition box 1 may further directly transmit a received target measurement signal to the department-level workstation device and the hospital-level data center/hospital-level emergency center management device by means of the far field communication module 302, so that the department-level workstation device and the hospital-level data center/hospital-level emergency center management device can obtain an analysis result by analysis and display corresponding analysis result data. Thus, medical care personnel at the department-level workstation device and the hospital-level data center/hospital-level emergency center management device can view the data.

Obviously, in other embodiment, both the wireless communication module 30 of the acquisition box 1 and the wireless communication module 201 of the monitoring device 2 may include a near field communication module and a far field communication module.

Referring to FIG. 13, it is a schematic diagram of the monitoring apparatus 200 worn on a subject. In some embodiments, the monitoring device 2 included in the monitoring apparatus 200 is a wearable monitoring device, which may be, for example, a wristband type monitoring device, a head-mounted monitoring device, etc. As shown in FIG. 12, the monitoring device 2 is a wristband type monitoring device that is worn on the wrist of the subject. The acquisition box 1 is connected to the skin of the subject by means of the measurement wire 3, and an outer surface of the housing 101 of the acquisition box 1 may be provided with a viscous material and may be adhered to the skin of the subject to achieve stable wearing of the acquisition box 1.

In some embodiments, an outer surface of the housing 101 of the acquisition box 1 may be provided with at least one suction cup by which the acquisition box may be sucked to the skin of the subject to achieve stable wearing of the acquisition box 1. Since the acquisition box 1 and the monitoring device 2 are connected in a wireless communication mode, inconvenience in wearing caused by wireless connection is avoided, and user experience is improved.

Thus, according to the acquisition box 1, the acquisition box assembly 100 and the monitoring apparatus 200 of the disclosure, the target measurement signal is obtained by designing an acquisition box 1 independent of the monitoring device 2 to at least reduce the voltage of the measurement signal received by the signal receiving end 10, achieving a defibrillation protection function; the size of the target monitoring device 2 may be kept small, and the target measurement signal is transmitted to the target monitoring device 2 in a wireless mode, avoiding the constraint of wired transmission, meeting the ECG measurement requirement and also bringing convenience for a user.

In the embodiments described above, particular emphasis is laid on the description of the embodiments, if there is not a detailed portion in an embodiment, reference may be made to relevant description of other embodiments, and different examples of a component in an embodiment, which are applicable to other embodiments, may be applied in other embodiments.

The embodiments of the disclosure have been described in detail above, and specific examples are used herein to explain the principles and implementation of the disclosure. The above description of the embodiments is only used to facilitate understanding of the method of the disclosure and the core concept thereof. Moreover, for those of ordinary skill in the art, there may be modifications in the specific implementation and application scope based on the concept of the disclosure. To sum up, the content of this specification should not be construed as limiting the disclosure.

## Claims

**1.** An acquisition box, **characterized in that**, the acquisition box comprises:
a signal receiving end configured to receive a measurement signal acquired from a subject;
a voltage processing module configured to reduce a voltage of the measurement signal received by the signal receiving end to obtain a target measurement signal; and
a wireless communication module configured to transmit the target measurement signal to a target monitoring device in a wireless mode.

**2.** The acquisition box of claim 1, **characterized in that**, the voltage processing module comprises an anti-defibrillation resistor module, the anti-defibrillation resistor module comprises an anti-defibrillation resistor, the anti-defibrillation resistor is connected to the signal receiving end, and the measurement signal received by the signal receiving end passes through the anti-defibrillation resistor to reduce the voltage.

**3.** The acquisition box of claim 2, **characterized in that**, the signal receiving end is configured to receive a plurality of the measurement signals, the anti-defibrillation resistor module comprises a plurality of groups of the anti-defibrillation resistor(s), and each of the measurement signals passes through a corresponding group of the anti-defibrillation resistor(s) to reduce the voltage.

**4.** The acquisition box of claim 3, **characterized in that**, each group of the anti-defibrillation resistor(s) comprises a plurality of anti-defibrillation resistors connected in series, and different groups of the anti-defibrillation resistor(s) comprise a same number or different number of the anti-defibrillation resistor(s).

**5.** The acquisition box of claim 3, **characterized in that**, the acquisition box further comprises a signal processing module, the signal processing module is coupled to the voltage processing module and configured to perform analog-to-digital conversion on the measurement signal with the voltage reduced by the voltage processing module, and the target measurement signal is a digital measurement signal that has further undergone the analog-to-digital conversion.

**6.** The acquisition box of claim 5, **characterized in that**, the acquisition box further comprises a circuit board, the voltage processing module is disposed in a first region of the circuit board, the signal processing module is disposed in a second region of the circuit board, and the first region is located between the second region and the signal receiving end.

**7.** The acquisition box of claim 6, **characterized in that**, the circuit board comprises two faces opposite to each other, a group of elements of the voltage processing module are disposed on one face of the first region of the circuit board, another group of elements of the voltage processing module are disposed on the other face of the first region of the circuit board, and the signal processing module is disposed on at least one face of the second region of the circuit board.

**8.** The acquisition box of claim 6, **characterized in that**, the voltage processing module and the signal processing module are disposed on a same face of the circuit board.

**9.** The acquisition box of claim 6, **characterized in that**, the acquisition box further comprises at least two circuit boards, the at least two circuit boards are disposed in a stacked manner, a group of elements of the voltage processing module and a group of elements of the signal processing module are respectively disposed in the first region and the second region of one circuit board, and another group of elements of the voltage processing module and another group of elements of the signal processing module are respectively disposed in the first region and the second region of another circuit board.

**10.** The acquisition box of claim 9, **characterized in that**, each of the circuit boards is provided with elements of the signal processing module and elements of the voltage processing module on at least one face, and at least one of the circuit boards is provided with elements of the voltage processing module on two faces.

**11.** The acquisition box of claim 9 or 10, **characterized in that**, an insulating material is disposed between two adjacent circuit boards of the at least two circuit boards.

**12.** The acquisition box of claim 8 or 9, **characterized in that**, adjacent groups of the anti-defibrillation resistor(s) disposed on a same face of a same circuit board are isolated.

**13.** The acquisition box of claim 12, **characterized in that**, a region between the adjacent groups of the anti-defibrillation resistor(s) disposed on the same face of the same circuit board is carved out, and/or a region between the adjacent groups of the anti-defibrillation resistor(s) disposed on the same face of the same circuit board is provided with an isolation wall to isolate the adjacent groups of the anti-defibrillation resistor(s).

**14.** The acquisition box of claim 5, **characterized in that**, the acquisition box further comprises at least two circuit boards, the at least two circuit boards are flatly laid, and the voltage processing module and the signal processing module are disposed on different circuit boards.

**15.** The acquisition box of claim 3, **characterized in that**, the acquisition box further comprises a clamping circuit, and the clamping circuit is connected to an output end of the anti-defibrillation resistor module for clamping the voltage of the measurement signal to a preset voltage when the voltage of the measurement signal reduced by passing through the anti-defibrillation resistor module is higher than a trigger voltage.

**16.** The acquisition box of claim 15, **characterized in that**, the clamping circuit comprises a plurality of clamping diodes, a negative pole of each of the clamping diodes is connected to an end, away from the signal receiving end, of a group of the anti-defibrillation resistor(s), and positive poles of all clamping diodes are electrically connected to one another.

**17.** The acquisition box of claim 3, **characterized in that**, the signal receiving end is configured to be connected to a measurement wire to receive the measurement signal acquired by the measurement wire from the subject.

**18.** The acquisition box of claim 17, **characterized in that**, the measurement wire is a bifurcated measurement wire or a straight measurement wire, and comprises a plurality of measurement ends configured to respectively acquire a plurality of measurement signals from different parts of the subject.

**20.** The acquisition box of claim 18, **characterized in that**, the signal receiving end is detachably or fixedly connected to the measurement wire.

**21.** The acquisition box of claim 1, **characterized in that**, the target monitoring device comprises at least one of a wearable monitoring device, a bedside monitoring device, a department-level workstation device, and a hospital-level data center/hospital-level emergency center management device.

**22.** A monitoring apparatus, **characterized in that**, the monitoring apparatus comprises: a monitoring device and the acquisition box as claimed in any one of claims 1-21, wherein the monitoring device comprises a main case and a control module disposed within the main case, and the monitoring device is connected to the acquisition box in a wireless communication mode.

**23.** The monitoring apparatus of claim 26, **characterized in that**, the monitoring device is a wearable monitoring device.
